# EUROPÄISCHE PATENTSCHRIFT

(11) **EP 0 993 308 B1**
(45) Veröffentlichungstag und Bekanntmachung des Hinweises auf die Patenterteilung: **21.07.2004**
(21) Anmeldenummer: 98936352.8
(22) Anmeldetag: 18.06.1998
(51) Int. Cl.: A61L 31/00

(54) **MIT FLUORALKYLGRUPPEN BESCHICHTETE STENTS, VERFAHREN ZU IHRER HERSTELLUNG UND IHRE VERWENDUNG ZUR RESTENOSEPROPHYLAXE**
STENTS COATED WITH FLUOROALKYL GROUPS, METHOD FOR PRODUCING SAME AND THE USE THEREOF IN THE PROPHYLAXIS OF RESTENOSIS
EXTENSEURS RECOUVERTS DE GROUPES FLUOROALKYLE, LEUR PROCEDE DE PRODUCTION ET LEUR UTILISATION POUR LA PROPHYLAXIE DE LA RESTENOSE

(30) Priorität: 24.06.1997 DE 19727838
(43) Veröffentlichungstag der Anmeldung: 19.04.2000
(73) Patentinhaber: SCHERING AKTIENGESELLSCHAFT, 13342 Berlin (DE)
(72) Erfinder: KRAUSE, Werner, D-13505 Berlin (DE); HÖCKER, Hartwig, D-52076 Aachen (DE); KLEE, Doris, D-52074 Aachen (DE); LAHANN, Jörg, D-66571 Habach (DE)
(86) Internationale Anmeldenummer: PCT/EP1998/003627
(87) Internationale Veröffentlichungsnummer: WO 1998/058680

(56) Entgegenhaltungen:
- EP-A- 0 519 087
- DE-A- 3 918 736
- DE-U- 29 711 398
- US-A- 5 607 475

## Beschreibung

Die Erfindung liegt auf dem Gebiet der Gefäßimplantate und beschreibt mit Fluoralkylgruppen beschichtete Stents, Verfahren zu ihrer Herstellung und ihre Verwendung zur Restenoseprophylaxe.

### Stand der Technik

Stents sind Stand der Technik (Pschyrembel, Klinisches Wörterbuch 257. Auflage, Verlag W. de Gruyter). Stents sind Endoprothesen, die die Offenhaltung gangartiger Strukturen in Körpern von Menschen oder Tieren ermöglichen (z.B. Gefäß-, Ösophagus-, Trachea-, Gallengangstent). Sie werden als palliative Maßnahme bei Verengungen durch Verschluß (z.B. Atherosklerose) oder Druck von außen (z.B. bei Tumoren) verwendet. Radioaktive Stents werden beispielsweise nach gefäßchirurgischen oder interventionell radiologischen Eingriffen (z.B. Ballonangioplastie) zur Restenoseprophylaxe eingesetzt.

Es besteht nun das Problem, daß der Stent für den Körper einen Fremdkörper darstellt und es zu Unverträglichkeitsreaktionen kommt.

Aufgabe der vorliegenden Erfindung ist es daher, Stents zur Verfügung zu stellen, die besser verträglich sind als herkömmliche Stents. Diese Aufgabe wird durch die nachfolgend beschriebenen Stents gelöst, wie sie in den Patentansprüchen gekennzeichnet sind.

### Beschreibung der Erfindung

Die oben geschilderte Aufgabe wird erfindungsgemäß dadurch gelöst, daß die Oberfläche der Stents mit einem Trägerpolymer beschichtet wird, von dem Fluoralkylgruppen bürstenförmig abstehen.

Die erfindungsgemäße Vorrichtung besteht somit aus dem Stentgrundkörper, der mit einem Trägerpolymer beschichtet ist, an das Perfluoralkylketten gebunden sind, die bürstenförmig von der Stentoberfläche abstehen.

Als Grundkörper können die handelsüblichen Gefäßimplantante verwendet werden, z.B. ein Wiktor-Stent, ein Strecker-Stent, ein Nitinol-Stent oder ein Palmaz-Schatz Stent. Der Stentgrundkörper kann metallisch oder aus einem Polymer hergestellt sein.

Als Trägerpolymere kommen beispielsweise modifizierte Polyurethane in Betracht, die derivatisierbare Gruppen tragen, z.B. Amino-, Hydroxyl-, Carboxyl-, Carbony-, Thiol-, Thiocarboxyl- oder andere Funktionen, die umgesetzt werden können. Die derivatisierbaren Gruppen können auch über Polyethylenglycole, Polysaccharide, Cyclodextrine, Polyaminopolycarbonsäuren oder Proteine im Trägerpolymer enthalten sein.

Es sind aber auch Polymere auf der Basis von Polyamino-p-xylylen (Formel I) vorteilhaft als Trägerpolymere einsetzbar.

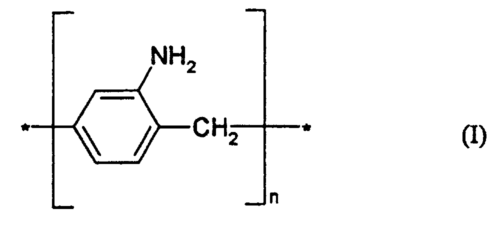

Weiter können folgende Polymere als Trägerpolymere verwendet werden:
Polyorganosilane, Polyvinylpyrrolidone, Polymethylmethacrylate, Polyhydroxymethylmethacrylate, Mischpolymere aus N-Vinylpyrrolidon und Hydroxymethylmethacrylat, Polyamide, Polyacrylamide, Polyethylene, Polyethylenoxide, Polyethylenglycole, Polyester, Polypropylenoxide, Polysiloxane, PVC-Derivate, Polyvinyllactame, Polyethylenterephthalate, Polysilicone, Polysaccharide, Proteine, Polysulfone oder Polysulfonate, mit der Maßgabe, daß sie eine oder mehrere der oben genannten derivatisierbaren Gruppen enthalten.

An das Trägerpolymer sind fluoralkylkettenhaltige Moleküle der allgemeinen Formel II gebunden:

(X-Y-(CF₂)ₙ-Z)ₘ (II)

Dabei bedeuten
- X: eine direkte Bindung zum Trägerpolymer, eine Amino-, Ether-, Thioether-, Carbonyl-, Thiocarbonyl-, Sulfonyl-, Sulfuryl- oder Phosphorylgruppe oder eine längere Brücke, z.B. eine Alkylkette, die durch Heteratome unterbrochen sein kann und/oder durch Heteroatome substituiert sein kann, eine Aminosäure, Dicarbonsäure, ein Peptid, Nucleotid oder einen Zucker,
- Y: eine direkte Bindung, eine Amino-, Ether-, Thioether-, Carbonyl-, Thiocarbonyl-, Sulfonyl-, Sulfuryl- oder Phosphorylgruppe oder eine längere Brücke, z.B. eine Alkylkette, die durch Heteratome unterbrochen sein kann und/oder durch Heteroatome substituiert sein kann, eine Aminosäure, Dicarbonsäure, ein Peptid, Nucleotid oder einen Zucker,
- Z: ein Fluor- oder ein Wasserstoffatom,
- n: eine natürliche Zahl größer oder gleich 1,
- m: eine natürliche Zahl zwischen 1 und der Anzahl der derivatisierbaren Gruppen im Trägerpolymer.

Bevorzugt ist n größer als 5, besonders bevorzugt ist n größer als 10.

Die erfindungsgemäßen Stents können beispielhaft folgendermaßen hergestellt werden:
1. Ein unbeschichteter Stent kann zunächst mit einem Trägerpolymer (z.B. ein Polyurethan, erhältlich aus der Reaktion von 3,3'-Diacetylamino-diphenylmethan-4-4'-diisocyanat und Butandiol und nachfolgender Entfernung der Schutzgruppen) beschichtet werden. Dieses Polymer ist derartig modifiziert, daß es derivatisierbare Gruppen trägt (in diesem Beispiel Aminogruppen). Das Polymer wird in einem Lösemittel (z.B. Chloroform} gelöst und der Stent in die Polymerlösung eingetaucht. Nach Entnahme des Stents aus der Polymerlösung wird er in einer Trockenkammer bei Raumtemperatur getrocknet.
2. Alternativ zu 1. kann das Trägerpolymer mit Hilfe der Gasphasenabscheidung oder der Plasmapolymerisation auf den Stent aufgebracht werden. Dieses Verfahren beruht z.B. auf dem in der deutschen Offenlegungsschrift DE 196 04 173 A1 offenbarten Verfahren zur Erzeugung antithrombogener Oberflächen auf medizinischen Gegenständen. Bei diesem Verfahren wird ein funktionalisiertes Polymer durch Gasphasenbeschichtung bei erhöhten Temperaturen und reduzierten Drücken auf den metallischen Stentgrundkörper aufgebracht.
3. Der nach 1. oder 2. beschichtete Stent wird mit einer Lösung des Derivatisierungsmittels - wie nachfolgend beschrieben - versetzt.

Die Derivatisierung erfolgt durch Reaktion der Gruppen XH (X bedeutet dabei eine derivatisierbare Funktion, z.B. eine Amino-, Hydroxyl- oder Thiolgruppe) der mit dem Trägerpolymer beschichteteten Stents (Polymer-XH) mit Verbindungen der allgemeinen Formel III

Nu-CO-L-R^{F} (III)

worin
- R^{F}: eine Fluoralkylkette darstellt,
- L: eine direkte Bindung, eine Alkylgruppe, die durch Heteroatome unterbrochen und/oder substituiert sein kann, eine Aminosäure, ein Peptid, Nucleotid oder ein Zucker sein kann,
- Nu: in der Bedeutung eines Nucleofugs steht.

Falls die Reste L Hydroxylgruppen enthalten, können sie ggf. durch Acetyl- oder Isopropylidengruppen geschützt werden. Die Schutzgruppentechnik ist dem Fachmann vertraut.

Als Nucleofug dienen vorteilhafterweise die Reste:
I, Br, Cl, F, ―OTs, ―OMs,

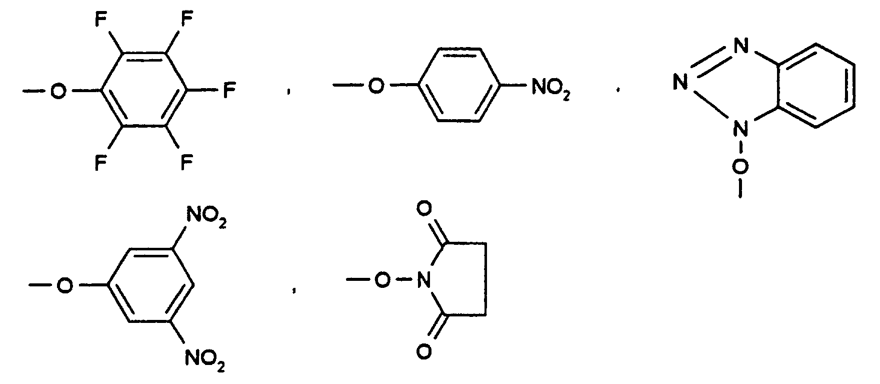

Die Umsetzung wird im Gemisch von Wasser und organischen Lösungsmitteln wie: Isopropanol, Ethanol, Methanol, Butanol, Dioxan, Tetrahydrofuran, Dimethylformamid, Dimethylacetamid, Formamid oder Dichlormethan durchgeführt. Bevorzugt sind ternäre Gemische aus Wasser, Isopropanol und Dichlormethan.

Die Umsetzung wird in einem Temperaturintervall zwischen -10°C - 100°C, vorzugsweise zwischen 0°C - 30°C durchgeführt.

Als Säurefänger dienen anorganische und organische Basen wie Triethylamin, Pyridin, N-Methylmorpholin, Diisopropylethylamin, Dimethylaminopyridin, Alkali- und Erdalkalihydroxyde, ihre Carbonate oder Hydrogencarbonate wie Lithiumhydroxid, Natriumhydroxid, Kaliumhydroxid, Natriumcarbonat, Natriumhydrogencarbonat, Kaliumhydrogencarbonat.

Die Verbindungen der allgemeinen Formel III werden aus Verbindungen der allgemeinen Formel IV erhalten:

HO₂C-L-R^{F} (IV),

in der
R^{F}, L die oben genannte Bedeutung haben, nach den dem Fachmann allgemein bekannten Verfahren der Säureaktivierung wie:
durch Umsetzung der Säure mit Dicyclohexylcarbodiimid, N-Hydroxysuccinimid/Dicyclohexylcarbodiimid, Carbonyldiimidazol, 2-Ethoxy-1-ethoxycarbonyl-1,2-dihydrochinolin, Oxalsäuredichlorid oder Chlorameisensäurisobutylester in der in der Literatur beschriebenen Weise erfolgen:
◆ Aktivierung von Carbonsäuren. Übersicht in Houben-Weyl, Methoden der Organischen Chemie, Band XV/2, Georg Thieme Verlag Stuttgart, 19.
◆ Aktivierung mit Carbodiimiden. R. Schwyzer u. H. Kappeler, Helv. 46:1550 (1963).
◆ E. Wünsch et al., B. 100:173 (1967).
◆ Aktivierung mit Carbodiimiden/Hydroxysuccinimid: J. Am. Chem. Soc. 86:1839 (1964) sowie J. Org. Chem. 53:3583 (1988). Synthesis 453 (1972).
◆ Anhydridmethode, 2-Ethoxy-1-ethoxycarbonyl-1,2-dihydrochinolin: B. Belleau et al., J. Am. Chem. Soc., 90:1651 (1986), H. Kunz et al., Int. J. Pept. Prot. Res., 26:493 (1985) und J. R. Voughn, Am. Soc. 73:3547 (1951).
◆ Imidazolid-Methode: B.F. Gisin, R.B. Menifield, D.C. Tosteon, Am. Soc. 91:2691 (1969).
◆ Säurechlorid-Methoden, Thionylchlorid: Helv., 42:1653 (1959).
◆ Oxalylchlorid: J. Org. Chem., 29:843 (1964).

Die Verbindungen der allgemeinen Formel IV sind Kaufware (Fluorochem, ABCR) oder werden aus Verbindungen der allgemeinen Formel V

H-Q-L-R^{F} (V)

mit
- Q: in der Bedeutung von
― O ― oder ― S ― oder 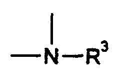 oder 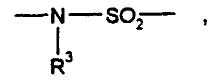 mit einer Bindung vom Stickstoffatom zum Wasserstoffatom, oder 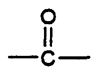 durch Umsetzen mit Verbindungen der allgemeinen Formel VI

Hal-CH₂-CO-OR¹ (VI)
mit
- Hal: in der Bedeutung, Cl, Br, I und
- R¹: in der Bedeutung von H, Methyl, Ethyl, t-Butyl, Benzyl, Isopropyl, dargestellt beispielsweise nach C.F. Ward, Soc. 121, 1161 (1922),
nach den dem Fachmann bekannten Methoden wie Alkylierung von Alkoholen mit Alkylhalogeniden [Houben-Weyl, Methoden der Organischen Chemie, Sauerstoffverbindungen I, Teil 3, Methoden zur Herstellung und Umwandlung von Ethern, Georg Thieme Verlag, Stuttgart 1965, Alkylierung von Alkoholen mit Alkylhalogeniden S. 24, Alkylierung von Alkoholen mit Alkylsulfaten S. 33] oder N-Alkylierung eines Sulfonamids mit Alkylsulfonaten [Houben-Weyl, Methoden der organischen Chemie, XI/2 Stickstoffverbindungen, Georg Thieme Verlag Stuttgart, 1957, S. 680; J.E. Rickman and T. Atkins, Am. Chem. Soc., 96:2268, 1974, 96:2268; F. Chavez and A.D. Sherry, J. Org. Chem. 1989, 54:2990] erhalten.
Für den Fall daß Q die Gruppe 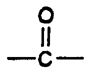 bedeutet, wird die Umsetzung mit einem Wittig-Reagenz der Struktur 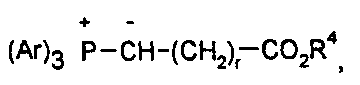 wobei, r die Zahlen 0 - 16 bedeutet, vorgenommen. Die dabei entstandene - CH=CH-Doppelbindung kann als Bestandteil der Struktur erhalten bleiben oder durch katalytische Hydrierung (Pd 5 %/C) in eine -CH₂-CH₂-Gruppierung überführt werden.

Die Verbindungen der allgemeinen Formel VI sind Kaufware (Fluorochem, ABCR).

Die oben beschriebenen Verfahren werden im allgemeinen bei Temperaturen von 0-80°C durchgeführt. Bei der Beschichtung des Stents mit dem Polymer können in Abhängigkeit von dem jeweiligen Polymer Lösemittel eingesetzt werden. Bei Einsatz eines nichtwäßrigen Lösemittels soll dieses vor der Implantation entfernt werden.

Analog erfolgt die Derivatisierung der Gruppen XH (X bedeutet dabei eine Carboxylgruppe) der polymerbeschichteteten Stents (Polymer-COOH) mit Verbindungen der allgemeinen Formel VII

H₂N-L-R^{F} (VII)

durch Aktivierung der COOH-Gruppen des Polymers wie oben beschrieben. L und R^{F} haben dabei die oben beschriebene Bedeutung.

Die nötigen Arbeitsgänge zur Durchführung der oben prinzipiell beschriebenen Verfahren sind dem Fachmann bekannt. Spezielle Ausführungsformen sind detailliert in den Beispielen beschrieben.

Die erfindungsgemäßen Stents lösen die eingangs beschriebene Aufgabe. Die erfindungsgemäßen Stents sind physiologisch gut verträglich.

Die Oberflächenbeschichtung der erfindungsgemäßen Stents, wie sie oben beschrieben wurde, läßt sich auch allgemein bei der Beschichtung von Oberflächen anwenden, um diese inert zu machen. Dies gilt besonders für medizinische Anwendungen, z.B. für Katheter, Sonden, Dialysegeräte, künstliche Herzklappen, Prothesen usw.

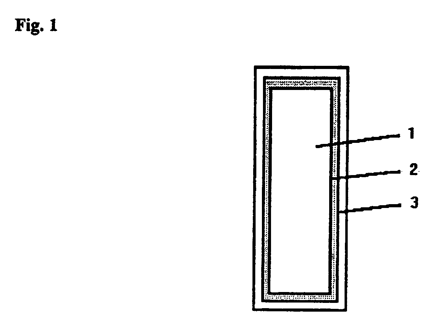

Fig. 1 zeigt schematisch den Aufbau der erfindungsgemäßen Stents. Darin bedeuten
1: Stentgrundkörper
2: Trägerpolymer
3: die fluoralkylkettentragende Schicht.

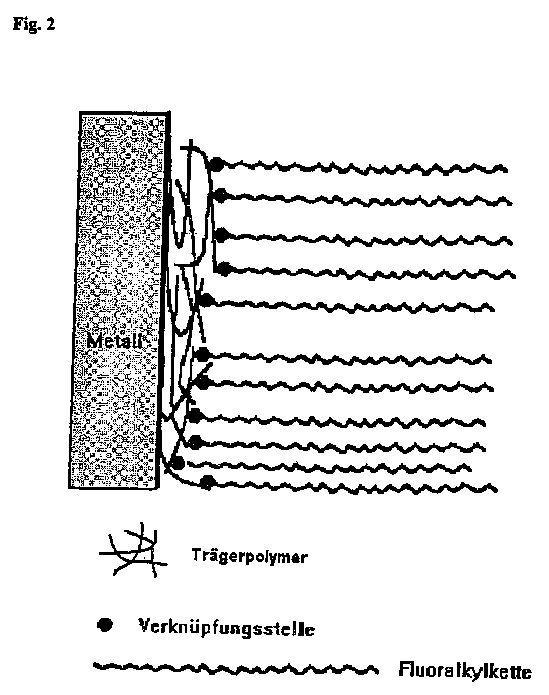

Fig. 2 ist eine Darstellung, bei der die bürstenartige Struktur der fluoralkylkettentragenden Schicht schematisch aufgezeigt wird.

### Ausführungsbeispiele:

Die folgenden Beispiele sollen den Erfindungsgegenstand erläutern, ohne ihn auf diese beschränken zu wollen.

### Beispiel 1

Als Trägerpolymer wird Polyurethan verwendet, das durch Reaktion von 3,3'-Diacetylamino-diphenylmethan-4,4'-diisocyanat und Butandiol erhältlich ist. Nach der Polymerisation werden die Acetylschutzgruppen entfernt. Die Stents werden dadurch beschichtet, daß sie in eine 5%ige Chloroform-Lösung des Polymers eingetaucht werden. Danach läßt man sie einer Reinraum-Trockenkammer bei Zimmertemperatur trocknen. Die durchschnittliche Schichtdicke beträgt 20 µm. Die Derivatisierung mit Fluoralkylgruppen erfolgt durch Umsetzung freier Aminogruppen mit dem Säurechlorid der Formel VIII

Cl-CO-(CF₂)₁₄-CF₃ (VIII),

wie es in der Literatur beschrieben und dem Fachmann geläufig ist. Nach der Trockung ist der Stent gebrauchsfertig.

### Beispiel 2

Die Beschichtung des Stents mit einem Polymer, das freie Carboxylgruppen an der Oberfläche trägt, erfolgt wie unter Beispiel 1 beschrieben. Danach erfolgt die Umsetzung mit Thionylchlorid zu einem Saurechlorid, wie es dem Fachmann bekannt ist. Anschließend werden die Chloratome des Säurechlorids mit einem Amin der Formel IX umgesetzt.

H₂N-(CF₂)₁₆-CF₃ (IX)

Nach der Trockung ist der Stent gebrauchsfertig.

### Beispiel 3

Die Beschichtung eines Metallstents durch CVD-Polymerisation (CVD: Chemical Vapour Deposition) von 4-Amino-[2.2]-paracyclophan erfolgt in einer geeignet konzipierten Anlage. Die Anlage ist mit einer Argonbombe verbunden, da Argon als Trägergas fungiert. Die Argonzuleitung ist mit einem 380 mm langen Quarzglasrohr mit einem Außendurchmesser von 30 mm verbunden. Das Quarzglasrohr ist an seinem anderen Ende mit einem Edelstahlrezipienten verbunden. Das Quarzglasrohr ist frei schwebend in einem Dreizonenröhrenofen gelagert, der eine beheizte Länge von 320 mm und einen Innendurchmesser von 32 mm besitzt. Alle drei Heizzonen lassen sich bis 800°C erhitzen. Der zu beschichtende Stent wird über das abnehmbare Schauglas auf dem Probenhalter fixiert. Anschließend wird der Reaktor wieder verschlossen und die Anlage wird durch Betätigung des Hauptschalters in Betrieb genommen. Gleichzeitig werden die beiden Kühlkreisläufe aktiviert, und die Rezipientenwand wird auf 100°C geheizt. Dann wird ein Porzellanschiffchen mit einer eingewogenen Menge an Monomer in die Sublimationszone gestellt und diese wieder verschlossen. Der Reaktor wird dann auf einen Basisdruck von 0.03 mbar abgepumpt. Nun wird ein Trägergasstrom von 20 sccm eingestellt und anschließend ein Arbeitsdruck von 0.2 mbar vorgegeben. Man wartet nun so lange, bis sowohl der Trägergasfluß als auch der Arbeitsdruck konstant sind. Nun gibt man die gewünschte Pyrolysetemperatur von 680°C vor und wartet, bis diese Temperatur in der Pyrolysezone erreicht wird. Dann läßt man den Probenhalter mit einer Drehgeschwindigkeit von 20 U/min rotieren und erhitzt die Sublimationszone auf 290°C. Der Beschichtungsprozeß wird mit Hilfe des Schichtdickenmonitors verifiziert. Wenn die gewünschte Schichtdicke von 280 nm erreicht ist, kann der Beschichtungsprozeß beendet werden. Dazu werden die Ofenregler, der Drehmotor des Probenhalters und der Trägergasstrom ausgeschaltet, das Drosselventil geöffnet und noch einmal aufBasisdruck abgepumpt. Anschließend wird die Pumpe abgeschaltet, die Anlage über das Belüftungsventil belüftet und die Probe entnommen.

Die Derivatisierung mit Fluoralkylgruppen erfolgt wie in Beispiel 1 durch Umsetzung der freien Aminogruppen auf dem Trägerpolymer mit dem Säurechlorid der Formel VIII

Cl-CO-(CF₂)₁₄-CF₃ (VIII),

wie es in der Literatur beschrieben und dem Fachmann geläufig ist. Nach der Trockung ist der Stent gebrauchsfertig.

## Patentansprüche

1. Stent, **dadurch gekennzeichnet, daß** er aus einem Stentgrundkörper besteht, der mit einem Trägerpolymer beschichtet ist, an das Perfluoralkylketten gebunden sind, die bürstenförmig von der Stentoberfläche abstehen.

2. Stent gemäß Anspruch 1, **dadurch gekennzeichnet, daß** der Stentgrundkörper ein metallischer Stentgrundkörper oder ein aus einem Polymer hergestellter Stent ist.

3. Stent gemäß Anspruch 2, **dadurch gekennzeichnet, daß** der metallische Stentgrundkörper ein Wiktor-Stent, ein Palmaz-Schatz-Stent, ein Strecker-Stent oder ein Nitinol-Stent ist.

4. Stent gemäß Anspruch 1, **dadurch gekennzeichnet, daß** das Trägerpolymer eines der folgenden Polymere ist: ein Polyurethanderivat, ein Polyamino-p-xylylenderivat, ein Polyorganosilan, ein Polyvinylpyrrolidon, ein Polymethylmethacrylat, ein Polyhydroxymethylmethacrylat, ein Mischpolymer aus N-Vinylpyrrolidon und Hydroxymethylmethacrylat, ein Polyamid, ein Polyacrylamid, ein Polyethylen, ein Polyethylenoxid, ein Polyethylenglycol, ein Polyester, ein Polypropylenoxid, ein Polysiloxan, ein PVC-Derivat, ein Polyvinyllactam, ein Polyethylenterephthalat, ein Polysilicon, ein Polysaccharid, ein Protein, ein Polysulfon oder ein Polysulfonat.

5. Stent gemäß Anspruch 1, **dadurch gekennzeichnet, daß** an das Trägerpolymer fluoralkylkettenhaltige Moleküle der allgemeinen Formel II gebunden sind:
(X-Y-(CF₂)ₙ -Z)ₘ (II)
worin
X für eine direkte Bindung zum Trägerpolymer, eine Amino-, Ether-, Thioether-, Carbonyl-, Thiocarbonyl-, Sulfonyl-, Sulfuryl- oder Phosphorylgruppe oder eine längere Brücke zum Trägerpolymer steht, wie z.B. eine Alkylkette, die durch Heteroatome unterbrochen sein kann und/oder durch Hetreoatome substituiert sein kann, eine Aminosäure, eine Dicarbonsäure, ein Peptid, Nucleotid oder ein Zucker,
Y für eine direkte Bindung, eine Amino-, Ether-, Thioether-, Carbonyl-, Thiocarbonyl-, Sulfonyl-, Sulfuryl- oder Phosphorylgruppe oder eine längere Brücke steht, wie z.B. eine Alkylkette, die durch Heteroatome unterbrochen sein kann und/oder durch Heteroatome substituiert sein kann, eine Aminosäure, eine Dicarbonsäure, ein Peptid, Nucleotid oder ein Zucker,
Z für ein Fluor- oder ein Wasserstoffatom steht,
n für eine natürliche Zahl größer oder gleich 1 steht,
m für eine natürliche Zahl zwischen 1 und der Anzahl der derivatisierbaren Gruppen im Trägerpolymer steht.

6. Verfahren zur Herstellung eines Stents gemäß einem der vorangegangenen Ansprüche, **dadurch gekennzeichnet, daß** ein Stentgrundkörper mit einem Trägerpolymer beschichtet wird und anschließend die Oberfläche mit perfluoralkylkettenhaltigen Molekülen derivatisiert wird.

7. Verfahren gemäß Anspruch 6, **dadurch gekennzeichnet, daß** das Trägerpolymer durch Gasphasenbeschichtung oder Plasmapolymerisation auf den Stentgrundkörper aufgebracht wird.

8. Oberflächenbeschichtung, **dadurch gekennzeichnet, daß** an ein Trägerpolymer Perfluoralkylketten gebunden sind, die bürstenförmig von der Oberfläche abstehen.

9. Oberflächenbeschichtung gemäß Anspruch 8, **dadurch gekennzeichnet, daß** an das Trägerpolymer fluoralkylkettenhaltige Moleküle der allgemeinen Formel II gebunden sind:
(X-Y-(CF₂)ₙ-Z)ₘ (II)
worin
X für eine direkte Bindung zum Trägerpolymer, eine Amino-, Ether-, Thioether-, Carbonyl-, Thiocarbonyl-, Sulfonyl-, Sulfuryl- oder Phosphorylgruppe oder eine längere Brücke zum Trägerpolymer steht, wie z.B. eine Alkylkette, die durch Heteroatome unterbrochen sein kann und/oder durch Hetreoatome substituiert sein kann, eine Aminosäure, eine Dicarbonsäure, ein Peptid, Nucleotid oder ein Zucker,
Y für eine direkte Bindung, eine Amino-, Ether-, Thioether-, Carbonyl-, Thiocarbonyl-, Sulfonyl-, Sulfuryl- oder Phosphorylgruppe oder eine längere Brücke steht, wie z.B. eine Alkylkette, die durch Heteroatome unterbrochen sein kann und/oder durch Heteroatome substituiert sein kann, eine Aminosäure, eine Dicarbonsäure, ein Peptid, Nucleotid oder ein Zucker,
Z für ein Fluor- oder ein Wasserstoffatom steht,
n für eine natürliche Zahl größer oder gleich 1 steht,
m für eine natürliche Zahl zwischen 1 und der Anzahl der derivatisierbaren Gruppen im Trägerpolymer steht.

## Claims

1. Stent, **characterized in that** it consists of a stent parent substance that is coated with a carrier polymer to which are connected perfluoroalkyl chains that project from the stent surface like a brush.

2. Stent according to claim 1, wherein the stent parent substance is a metallic stent parent substance or a stent that is produced from a polymer.

3. Stent according to claim 2, wherein the metallic stent parent substance is a Wiktor stent, a Palmaz-Schatz stent, a Strecker stent, or a Nitinol stent.

4. Stent according to claim 1, wherein the carrier polymer is one of the following polymers: a polyurethane derivative, a polyamino-p-xylylene derivative, a polyorganosilane, a polyvinylpyrrolidone, a polymethylmethacrylate, a polyhydroxymethylmethacrylate, a mixed polymer from N-vinylpyrrolidone and hydroxymethylmethacrylate, a polyamide, a polyacrylamide, a polyethylene, a polyethylene oxide, a polyethylene glycol, a polyester, a polypropylene oxide, a polysiloxane, a PVC derivative, a polyvinyllactam, a polyethylene terephthalate, a polysilicone, a polysaccharide, a protein, a polysulfone or a polysulfonate.

5. Stent according to claim 1, wherein fluoroalkyl-chain-containing molecules of general formula II are connected to the carrier polymer:
(X-Y-(CF₂)ₙ-Z)ₘ (II)
in which
X stands for a direct bond to the carrier polymer, an amino, ether, thioether, carbonyl, thiocarbonyl, sulfonyl, sulfuryl or phosphoryl group or a longer bridge to the carrier polymer, e.g., an alkyl chain, which can be interrupted by heteroatoms and/or can be substituted by heteroatoms, an amino acid, a dicarboxylic acid, a peptide, nucleotide or a sugar,
Y stands for a direct bond, an amino, ether, thioether, carbonyl, thiocarbonyl, sulfonyl, sulfuryl or phosphoryl group or a longer bridge such as, e.g., an alkyl chain, which can be interrupted by heteroatoms and/or can be substituted by heteroatoms, an amino acid, a dicarboxylic acid, a peptide, nucleotide or a sugar,
Z stands for a fluorine or a hydrogen atom,
n stands for a natural number that is greater than or equal to 1,
m stands for a natural number of between 1 and the number of groups that can be derivatized in the carrier polymer.

6. Process for the production of a stent according to one of the preceding claims, wherein a stent parent substance is coated with a carrier polymer, and then the surface is derivatized with perfluoroalkyl-chain-containing molecules.

7. Process according to claim 6, wherein the carrier polymer is placed on the stent parent substance by gas phase coating or plasma polymerization.

8. Surface coating, wherein perfluoralkyl chains that project from the surface like a brush are connected to a carrier polymer.

9. Surface coating according to claim 8, wherein fluoroalkyl-chain-containing molecules of general formula II are connected to the carrier polymer:
(X-Y- (CF₂)ₙ-Z)ₘ (II)
in which
X stands for a direct bond to the carrier polymer, an amino, ether, thioether, carbonyl, thiocarbonyl, sulfonyl, sulfuryl or phosphoryl group or a longer bridge to the carrier polymer, such as, e.g., an alkyl chain, which can be interrupted by heteroatoms and/or can be substituted by heteroatoms, an amino acid, a dicarboxylic acid, a peptide, nucleotide or a sugar,
Y stands for a direct bond, an amino, ether, thioether, carbonyl, thiocarbonyl, sulfonyl, sulfuryl or phosphoryl group or a longer bridge, such as, e.g., an alkyl chain, which can be interrupted by heteroatoms and/or can be substituted by heteroatoms, an amino acid, a dicarboxylic acid, a peptide, nucleotide or a sugar,
Z stands for a fluorine or a hydrogen atom,
n stands for a natural number that is greater than or equal to 1,
m stands for a natural number of between 1 and the number of groups that can be derivatized in the carrier polymer.

## Revendications

1. Endoprothèse coronaire (stent), **caractérisée en ce qu'**elle est constituée par un corps de base d'endoprothèse coronaire, qui est revêtu par un polymère support auquel sont liées des chaînes perfluoroalkyle qui s'écartent sous forme d'une brosse de la surface de l'endoprothèse coronaire.

2. Endoprothèse coronaire selon la revendication 1, **caractérisée en ce que** le corps de base de l'endoprothèse est un corps de base de l'endoprothèse coronaire métallique ou une endoprothèse coronaire réalisée à partir d'un polymère.

3. Endoprothèse coronaire selon la revendication 2, **caractérisée en ce que** le corps de base de l'endoprothèse coronaire métallique est une endoprothèse coronaire de Wiktor, de Palmaz-Schatz, de Strecker ou de Nitinol.

4. Endoprothêse coronaire selon la revendication 1, **caractérisée en ce que** le polymère support est un des polymères suivants : un dérivé de polyuréthanne, un dérivé de polyamine -p -xylylène, un polyorganosilane, une polyvinylpyrrolidone, un poly(méthacrylate de méthyle), un poly(méthacrylate d'hydroxyméthyle), un copolymère de N-vinylpyrrolidone et de méthacrylate d'hydroxyméthyle, un polyamide, un polyacrylamide, un polyéthylène, un poly(oxyde d'éthylène), un polyéthylèneglycol, un polyester, un poly(oxyde de propylène), un polysiloxane, un dérivé de PVC, un polyvinyllactame, un poly(téréphtalate d'éthylène), un polysilicone, un polysaccharide, une protéine, une polysulfone ou un polysulfonate.

5. Endoprothèse coronaire selon la revendication 1, **caractérisée en ce que** des molécules contenant des chaînes contenant des groupes fluoroalkyle de formule générale (II) sont liées au polymère support :
(X-Y- (CF₂) ₙ-Z)ₘ (II)
où
X représente une liaison directe au polymère support, un groupe amino, éther, thioéther, carbonyle, thiocarbonyle, sulfonyle, sulfuryle ou phosphoryle ou un pont plus long avec le polymère support, tel que par exemple une chaîne alkyle, qui peut être interrompue par des hétéroatomes et/ou substituée par des hétéroatomes, un acide aminé, un acide dicarboxylique, un peptide, un nucléotide ou un sucre,
Y représente une liaison directe, un groupe amino, éther, thioéther, carbonyle, thiocarbonyle, sulfonyle, sulfuryle ou phosphoryle ou un pont plus long, tel que par exemple une chaîne alkyle, qui peut être interrompue par des hétéroatomes et/ou substituée par des hétéroatomes, un acide aminé, un acide dicarboxylique un peptide, un nucléotide ou un sucre,
Z représente un atome de fluor ou d'hydrogène,
n représente un nombre naturel supérieur ou égal à 1,
m représente un nombre naturel entre 1 et le nombre de groupes dérivables dans le polymère support.

6. Procédé pour la préparation d'une endoprothèse coronaire selon l'une quelconque des revendications précédentes, **caractérisé en ce qu'**on recouvre un corps de base d'une endoprothèse coronaire avec un polymère support et qu'on dérive ensuite la surface avec des molécules contenant des chaînes perfluoroalkyle.

7. , Procédé selon la revendication 6, **caractérisé en ce que** le polymère support est appliqué par un revêtement en phase gazeuse ou une polymérisation à partir d'un plasma sur le corps de base de l'endoprothèse coronaire.

8. Revêtement de surface, **caractérisé en ce que** des chaînes perfluoroalkyle, qui s'écartent sous forme de brosse de la surface, sont liées sur un polymère support.

9. Revêtement de surface selon la revendication 8, **caractérisé en ce que** des molécules contenant des chaînes fluoroalkyle de formule générale (II) sont liées sur le polymère support :
(X-Y-(CF₂)ₙ-Z)ₘ (II)
où
X représente une liaison directe au polymère support, un groupe amino, éther, thioéther, carbonyle, thiocarbonyle, sulfonyle, sulfuryle ou phosphoryle ou un pont plus long avec le polymère support, tel que par exemple une chaîne alkyle, qui peut être interrompue par des hétéroatomes et/ou substituée par des hétéroatomes, un acide aminé, un acide dicarboxylique, un peptide, un nucléotide ou un sucre,
Y représente une liaison directe, un groupe amino, éther, thioéther, carbonyle, thiocarbonyle, sulfonyle, sulfuryle ou phosphoryle ou un pont plus long, tel que par exemple une chaîne alkyle, qui peut être interrompue par des hétéroatomes et/ou substituée par des hétéroatomes, un acide aminé, un acide dicarboxylique , un peptide, un nucléotide ou un sucre,
Z représente un atome de fluor ou d'hydrogène,
n représente un nombre naturel supérieur ou égal à 1,
m représente un nombre naturel entre 1 et le nombre de groupes dérivables dans le polymère support.
